# EUROPEAN PATENT APPLICATION

(11) **EP 3 593 912 A2**
(43) Date of publication of application: **15.01.2020**
(21) Application number: 19181344.3
(22) Date of filing: 19.06.2019
(51) Int. Cl.: B06B 1/06

(54) **ULTRASONIC PROBE AND MANUFACTURING METHOD THEREOF**

(30) Priority: 20.06.2018 KR 20180070806
(71) Applicant: Samsung Medison Co., Ltd., Hongcheon-gun, Gangwon-do, 25108 (KR)
(72) Inventor: KIM, Seung Hyun, 13533 Gyeonggi-do (KR); GU, Jin Ho, 13647 Gyeonggi-do (KR); KIM, Ji Su, 14309 Gyeonggi-do (KR)
(74) Representative: D'Halleweyn, Nele Veerle Trees Gertrudis

(57) **Abstract**

Disclosed are an ultrasonic probe for obtaining an ultrasonic image and a manufacturing method thereof. The ultrasonic probe includes a transducer layer including a piezoelectric layer configured to generate ultrasonic waves and an acoustic layer disposed below the piezoelectric layer, and a matching layer disposed above the piezoelectric layer, wherein the transducer layer includes an active portion configured to transmit and receive ultrasonic waves, and a stepped portion extending outward from the active portion to prevent cutting damage of the active portion.

## Description

### BACKGROUND

### 1. Technical Field

The disclosure relates to an ultrasonic probe for obtaining an ultrasonic image and a manufacturing method thereof.

### 2. Description of the Related Art

An ultrasonic imaging apparatus is an apparatus that irradiates an ultrasonic signal from a body surface of a target toward a target site in the body and obtains an image of a monolayer or blood flow of soft tissue without invasion by using information of a reflected ultrasonic signal (ultrasonic echo signal).

The ultrasonic imaging apparatus is small, inexpensive, real-time displayable, easy to use, and has a high level of safety because there is no radiation exposure, compared to other imaging diagnostic apparatuses such as an X-ray diagnostic apparatus, an X-ray CT scanner (Computerized Tomography Scanner), an MRI (Magnetic Resonance Image) and a nuclear medicine diagnostic apparatus.

Therefore, the ultrasonic imaging apparatus has been widely used for diagnosis of the heart, abdomen, urinary system and obstetrics.

Generally, the ultrasonic imaging apparatus may include a main body and an ultrasonic probe that transmits ultrasonic signals to a target object to be diagnosed and receives signals reflected from the target object.

The ultrasonic probe may have a structure in which an ultrasonic signal transmitted from an internal piezoelectric layer is transmitted to a target object through a lens provided so as to come into contact with the target object and an ultrasonic signal reflected back from the target object is received again through the lens.

In general, a piezoelectric material used for the piezoelectric layer of the ultrasonic probe may include lead zirconate titanate (PZT) ceramics. The performance of PZT ceramics may be improved, but the performance improvement may be limited.

To replace this, a single crystal may be used as the piezoelectric material used for the piezoelectric layer of the ultrasonic probe.

Compared with PZT ceramics, the single crystal may have a piezoelectric distortion of three times or more, a large electromechanical coupling coefficient, and excellent piezoelectric characteristics. Therefore, in recent years, there has been increasing tendency to use the single crystal as the piezoelectric material.

On the other hand, in cutting machining by rotation, a rotation shaft of a cutting machine may be accompanied by minute tremors, whereby cutting damage such as chipping or cracks may occur on a cut surface of the single crystal having a relatively high brittleness.

The cutting damage may deteriorate the performance of the ultrasonic probe and may cause aging defects or the like, which may deteriorate the reliability in the quality of the ultrasonic probe. In particular, when the single crystal is processed in a single state by a cutting machine, the frequency of cutting damage occurring in the single crystal may be increased.

### SUMMARY

It is an aspect of the disclosure to provide an improved ultrasonic probe to improve the reliability in the quality of an ultrasonic probe by preventing aging failure of the ultrasonic probe.

It is another aspect of the disclosure to provide an improved ultrasonic probe to use a single crystal as a piezoelectric material forming a piezoelectric layer.

It is another aspect of the disclosure to provide an improved ultrasonic probe to prevent cutting damage that may occur in a single crystal forming a piezoelectric layer.

Additional aspects of the disclosure will be set forth in part in the description which follows and, in part, will be obvious from the description, or may be learned by practice of the disclosure.

In accordance with an aspect of the disclosure, an ultrasonic probe includes a transducer layer including a piezoelectric layer configured to generate ultrasonic waves and an acoustic layer disposed below the piezoelectric layer, and a matching layer disposed above the piezoelectric layer, wherein the transducer layer includes an active portion configured to transmit and receive ultrasonic waves, and a stepped portion extending outward from the active portion to prevent cutting damage of the active portion.

The stepped portion may extend from the active portion in an azimuth direction.

The piezoelectric layer may include a single crystal.

The acoustic layer may include an acoustic reflector having a tungsten carbide.

The active portion may include a first active portion constituting the acoustic layer and a second active portion disposed above the first active portion to constitute the piezoelectric layer, and the stepped portion may be stepped with the first active portion.

The height of the stepped portion may be lower than the height of the first active portion.

The ultrasonic probe may further include an anti-vibration member covering an outer side surface of the active portion to protect the active portion and disposed on an upper portion of the stepped portion.

The anti-vibration member may have a hardness higher than the hardness of the piezoelectric layer.

The piezoelectric layer and the acoustic layer may be bonded by an adhesive, and the anti-vibration member may include a component of the adhesive.

The transducer layer may further include a damage portion disposed above the stepped portion to prevent cutting damage of the active portion.

The damage portion may be spaced apart from the outer side surface of the active portion to form a cut portion together with the active portion and the stepped portion, and the anti-vibration member may be received in the cut portion.

The damage portion may include a first damage portion extending upward from the stepped portion to constitute the acoustic layer and a second damage portion disposed above the first damage portion to constitute the piezoelectric layer.

The stepped portion may have a curvature.

The matching layer may have a width larger than the width of the active portion to cover the cut portion and the damage portion.

The matching layer may include a conductive matching layer, and the cut portion may extend from the inside of the acoustic layer to an upper end of the conductive matching layer.

In accordance with another aspect of the disclosure, an ultrasonic probe includes a piezoelectric layer generating ultrasonic waves and including a single crystal, an acoustic layer disposed below the piezoelectric layer and including a tungsten carbide, and a cut portion formed near an edge of the piezoelectric layer to prevent cutting damage of the piezoelectric layer.

The cut portion may extend toward the inside of the acoustic layer.

In accordance with another aspect of the disclosure, a method of manufacturing an ultrasonic probe includes providing a piezoelectric layer configured to generate ultrasonic waves, bonding an acoustic layer to a lower end of the piezoelectric layer by an adhesive, and forming a cut portion toward the inside of the acoustic layer from an upper end of the piezoelectric layer near an edge of the piezoelectric layer and the acoustic layer so that the edge of the piezoelectric layer damaged by cutting damage is removed.

The method of manufacturing the ultrasonic probe may further include disposing an anti-vibration member inside the cut portion to protect the piezoelectric layer constituting an inner side surface of the cut portion.

The piezoelectric layer may include a single crystal.

### BRIEF DESCRIPTION OF THE DRAWINGS

These and/or other aspects of the disclosure will become apparent and more readily appreciated from the following description of the embodiments, taken in conjunction with the accompanying drawings of which:
FIG. 1 is a view illustrating an ultrasonic imaging apparatus including an ultrasonic probe according to an embodiment of the disclosure;
FIG. 2 is a view schematically illustrating the inside of the ultrasonic probe according to an embodiment of the disclosure;
FIG. 3 is a cross-sectional view taken along line A-A' in FIG. 2;
FIG. 4 is an enlarged view of a portion of a transducer in the ultrasonic probe according to an embodiment of the disclosure;
FIG. 5 is an enlarged view of a portion of a transducer in an ultrasonic probe according to another embodiment of the disclosure;
FIG. 6 is an enlarged view of a portion of a transducer in an ultrasonic probe according to another embodiment of the disclosure;
FIG. 7 is an enlarged view of a portion of a transducer in an ultrasonic probe according to another embodiment of the disclosure;
FIG. 8 is an enlarged view of a portion of a transducer in an ultrasonic probe according to another embodiment of the disclosure;
FIG. 9 is an enlarged view of a portion of a transducer in an ultrasonic probe according to another embodiment of the disclosure; and
FIG. 10 is a flow chart for manufacturing a part of a transducer in the ultrasonic probe according to an embodiment of the disclosure.

### DETAILED DESCRIPTION

The embodiments described in the present specification and the configurations shown in the drawings are only examples of preferred embodiments of the disclosure, and various modifications may be made at the time of filing of the disclosure to replace the embodiments and drawings of the present specification.

Like reference numerals or signs in the respective drawings of the present specification represent parts or components that perform substantially the same functions.

The terms used in the present specification are for the purpose of describing the embodiments and are not intended to restrict and/or to limit the disclosure. For example, the singular expressions herein may include plural expressions, unless the context clearly dictates otherwise.

The terms "comprises" and "has" are intended to indicate that there are features, numbers, steps, operations, elements, parts, or combinations thereof described in the specification, and do not exclude the presence or addition of one or more other features, numbers, steps, operations, elements, parts, or combinations thereof.

It will be understood that, although the terms first, second, etc. may be used herein to describe various components, these components should not be limited by these terms. These terms are only used to distinguish one component from another.

For example, without departing from the scope of the disclosure, the first component may be referred to as a second component, and similarly, the second component may also be referred to as a first component.

The term "and/or" includes any combination of a plurality of related items or any one of a plurality of related items.

In this specification, the terms "front," "rear," "upper," "lower," "left," and "right" are defined with reference to the drawings, and the shape and position of each component are not limited by these terms.

Hereinafter, embodiments of the disclosure will be described in detail with reference to the accompanying drawings.

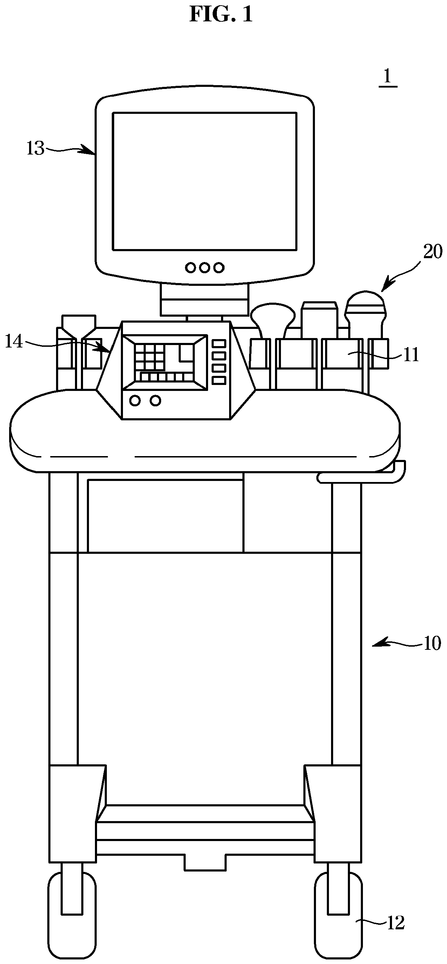

FIG. 1 is a view illustrating an ultrasonic imaging apparatus including an ultrasonic probe according to an embodiment of the disclosure.

As illustrated in FIG. 1, an ultrasonic imaging apparatus 1 according to the disclosure may include a main body 10 and an ultrasonic probe 20 that transmits ultrasonic signals to a target object to be diagnosed and receives signals reflected from the target object.

The ultrasonic probe 20 may transmit an ultrasonic signal to a target object to obtain an ultrasonic image, receive the ultrasonic signal reflected from the target object, and transmit the ultrasonic signal to a controller (not shown). The ultrasonic probe 20 may be connected to the main body 10 by a cable.

The main body 10 may be provided with a display 13 to display results of a diagnosis obtained through the received ultrasonic signal. An application related to the operation of the ultrasonic imaging apparatus 1 may be displayed on the display 13.

As an example, the display 13 may display an ultrasonic image obtained in an ultrasonic diagnostic process or items related to the operation of the ultrasonic imaging apparatus 1.

The display 13 may be implemented as a cathode ray tube (CRT), a liquid crystal display (LCD), or the like. A plurality of the displays 13 may be provided. In a case where the plurality of displays 13 are provided, the displays 13 may include a main display and a secondary display.

As an example, an ultrasonic image obtained in an ultrasonic diagnostic process may be displayed on the main display, and items related to the operation of the ultrasonic imaging apparatus 1 may be displayed on the secondary display.

The main body 10 may be provided with an input 14. The input 14 may be provided in the form of a keyboard, a foot switch, a foot pedal, or the like.

In a case where the input 14 is a keyboard, it may be provided at an upper portion of the main body 10. In a case where the input 14 is a foot switch or a foot pedal, it may be provided at a lower portion of the main body 10. An inspector may control the operation of the ultrasonic imaging apparatus 1 through the input 14.

The ultrasonic probe 20 may be mounted on the main body 10 by a holder 11. When the ultrasonic imaging apparatus 1 is not used, the inspector may mount the ultrasonic probe 20 on the holder 11 and store it in the holder 11.

The main body 10 may be provided with a moving device 12 to move the ultrasonic imaging apparatus 1. The moving device 12 may be a plurality of casters provided on the bottom surface of the main body 10.

The plurality of casters may be aligned to allow the main body 10 to travel in a specific direction, may be provided to be freely movable in any direction, or may be locked to stop at a specific position.

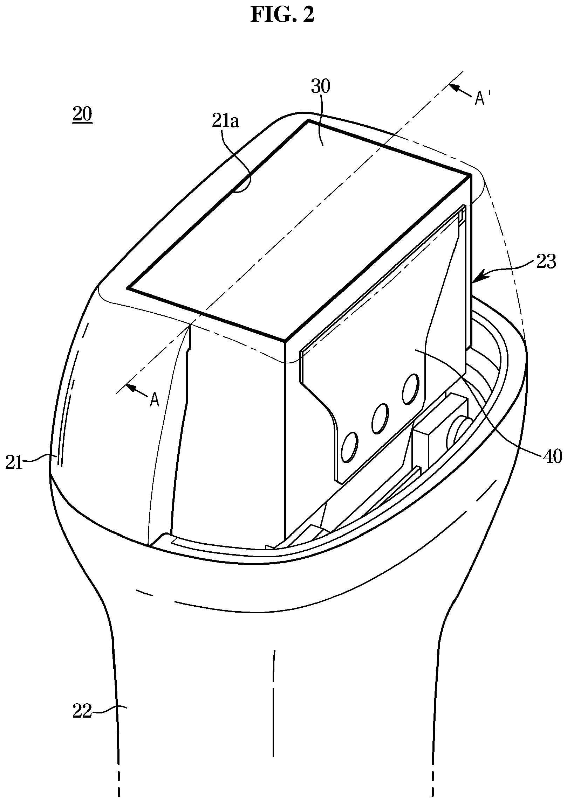

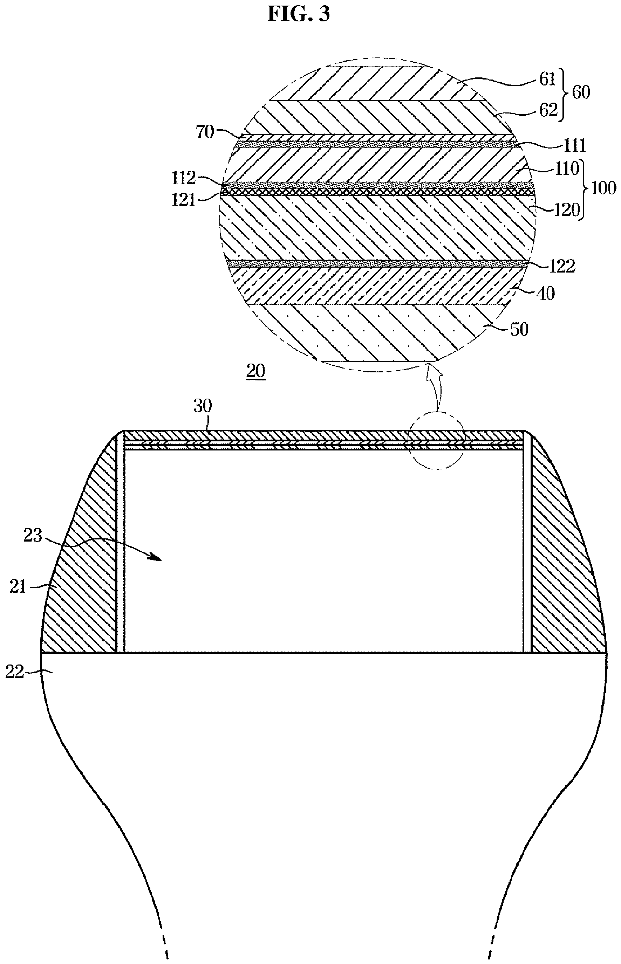

FIG. 2 is a view schematically illustrating the inside of the ultrasonic probe according to an embodiment of the disclosure, and FIG. 3 is a cross-sectional view taken along line A-A' in FIG. 2.

As illustrated in FIGS. 2 and 3, the ultrasonic probe 20 according to an embodiment of the disclosure may include a transducer 23 to generate an ultrasonic signal. The ultrasonic probe 20 may include a lens 30 provided to transmit the ultrasonic signal generated from the transducer 23 to the outside. The lens 30 may focus the ultrasonic signal.

The lens 30 may be provided with a material such as silicon, rubber, or the like having an acoustic impedance value similar to the acoustic impedance of the target object. The lens 30 may be a convex type having a convex curved surface at the center or may be a linear type having a flat surface.

The ultrasonic probe 20 may include a case 21 that accommodates the transducer 23 and has an opening 21a at one side so that the lens 30 is brought into contact with an external target object, and a handle 22 mounted on the other side of the case 21.

The transducer 23 may include a transducer layer 100, an acoustic matching layer 60 disposed in the front of the transducer layer 100, and a sound-absorbing layer 50 disposed in the rear of the transducer layer 100.

Generally, as a transducer, a magnetostrictive ultrasonic transducer utilizing the magnetostriction effect of a magnetic body, a capacitive micromachined ultrasonic transducer for transmitting and receiving ultrasonic waves by utilizing vibrations of hundreds or thousands of micro-machined thin films, or a piezoelectric ultrasonic transducer utilizing the piezoelectric effect of a piezoelectric material may be used.

Hereinafter, the piezoelectric ultrasonic transducer will be described as an embodiment of the transducer 23.

The effect of generating a voltage when mechanical pressure is applied to a given material and generating a mechanical deformation when a voltage is applied is called a piezoelectric effect and an inverse piezoelectric effect, and a material having such an effect may be referred to as a piezoelectric material.

That is, the piezoelectric material may include a material that converts electrical energy into mechanical vibration energy, and mechanical vibration energy into electrical energy.

The transducer layer 100 according to an embodiment of the disclosure may include a piezoelectric layer 110 made of a piezoelectric material that generates an ultrasonic wave by converting an electric signal into mechanical vibration when the electrical signal is applied, and an acoustic layer 120 disposed in the rear of the piezoelectric layer 110.

The piezoelectric layer 110 may be formed of zirconate titanate (PZT) ceramics, a PZNT single crystal made of a solid solution of zinc niobate and titanate, a PZMT single crystal made of a solid solution of magnesium niobate and titanate, or the like, which generates ultrasonic waves utilizing a resonance phenomenon.

By forming the piezoelectric layer 110 with a single crystal, the ultrasonic probe 20 having a wide band width may be formed, and ultrasonic signals in a high frequency range as well as a low frequency range may be transmitted and received.

Also, a process of making the divided acoustic modules into curvatures after channel division of the acoustic modules may be easily performed. Accordingly, the shape of the ultrasonic probe 20 may be not limited.

The piezoelectric layer 110 may be provided in the form of an air kerf to maximize ultrasonic generation performance.

The acoustic layer 120 may be provided to have an acoustic impedance higher than that of the piezoelectric layer 110. The acoustic layer 120 may be formed of a material having electrical conductivity.

The thickness of the acoustic layer 120 may be provided to be 1/2, 1/4, 1/8 or 1/16 of the wavelength of the piezoelectric material constituting the piezoelectric layer 110.

The acoustic layer 120 may include an acoustic reflector. The acoustic reflector may be disposed in the front of the sound-absorbing layer 50. The acoustic reflector may totally reflect the ultrasonic waves traveling to the sound-absorbing layer 50. Accordingly, the bandwidth of the ultrasonic probe 20 may be increased and the sensitivity may be increased.

The acoustic reflector may be made of a material having a very high acoustic impedance to totally reflect the ultrasonic waves. For example, the acoustic layer 120 may be made of at least one of a tungsten carbide and a graphite composite material.

Electrodes to which an electrical signal may be applied may be formed on the front and rear surfaces of the transducer layer 100. The electrodes may correspond to an anode and a cathode, respectively, and may be formed of a highly conductive metal such as gold, silver or copper.

A ground electrode 70 may be formed in the front of the transducer layer 100. A signal electrode 40 may be formed on the rear of the transducer layer 100. The ground electrode 70 and the signal electrode 40 each may be formed of a flexible printed circuit board (FPCB).

Electrodes may be formed on the front and rear surfaces of the piezoelectric layer 110. For example, a first electrode 111 connected to the ground electrode 70 may be provided on the front surface of the piezoelectric layer 110, and a second electrode 112 electrically connected to the signal electrode 40 may be provided on the rear surface of the piezoelectric layer 110.

However, the disclosure is not limited thereto, and the first electrode 111 formed on the front surface of the piezoelectric layer 110 may be connected to the signal electrode 40 and the second electrode 112 formed on the rear surface of the piezoelectric layer 110 may be connected to the ground electrode 70.

The acoustic layer 120 may include a third electrode 121 and a fourth electrode 122. The third electrode 121 may be formed on the front surface of the acoustic layer 120 and the fourth electrode 122 may be formed on the rear surface of the acoustic layer 120. The third electrode 121 and the fourth electrode 122 may be electrically connected.

The third electrode 121 and the fourth electrode 122 may be shorted. Although the third electrode 121 and the fourth electrode 122 are illustrated in FIG. 2, the disclosure is not limited thereto. Unlike the one illustrated in the figure, the acoustic layer 120 may not include the electrodes formed on the front and rear surfaces.

In this case, the acoustic layer 120 may be formed of a conductive material. When the acoustic layer 120 is formed of a conductive material, the acoustic layer 120 may be electrically connected to the second electrode 112 and the signal electrode 40 without the third electrode 121 and the fourth electrode 122.

The transducer 23 may include the matching layer 60. The matching layer 60 may serve to reduce the acoustic impedance difference between the piezoelectric layer 110 and the target object so that ultrasonic waves generated in the piezoelectric layer 110 are transmitted to the target object to the greatest extent possible.

The matching layer 60 reduces the acoustic impedance difference between the piezoelectric layer 110 and the target object to match the acoustic impedance of the piezoelectric layer 110 with the target object so that the ultrasonic waves generated in the piezoelectric layer 110 may be efficiently transmitted to the target object.

The matching layer 60 may be disposed to be adjacent to the piezoelectric layer 110. The matching layer 60 may be positioned in the front of the piezoelectric layer 110. The matching layer 60 may be provided to have an intermediate value between the acoustic impedance of the piezoelectric layer 110 and the acoustic impedance of the target object, and may be formed of glass or a resin material.

A plurality of the matching layers 60 having different materials may be provided to be laminated so that the acoustic impedance may gradually change from the piezoelectric layer 110 toward the target object. The matching layer 60 may include a first matching layer 61 and a second matching layer 62. The plurality of matching layers 60 may be provided to have different materials.

The transducer 23 may include the sound-absorbing layer 50. The sound-absorbing layer 50 may be disposed to be adjacent to the piezoelectric layer 110. The sound-absorbing layer 50 may be disposed in the rear of the piezoelectric layer 110.

The sound-absorbing layer 50 may reduce the pulse width of the ultrasonic waves by suppressing the free vibration of the piezoelectric layer 110 and prevent the ultrasonic image from being unnecessarily distorted by blocking the propagation of the ultrasonic wave to the rear of the piezoelectric layer 110.

The sound-absorbing layer 50 may be made of a material including rubber added with an epoxy resin, a tungsten powder, and the like.

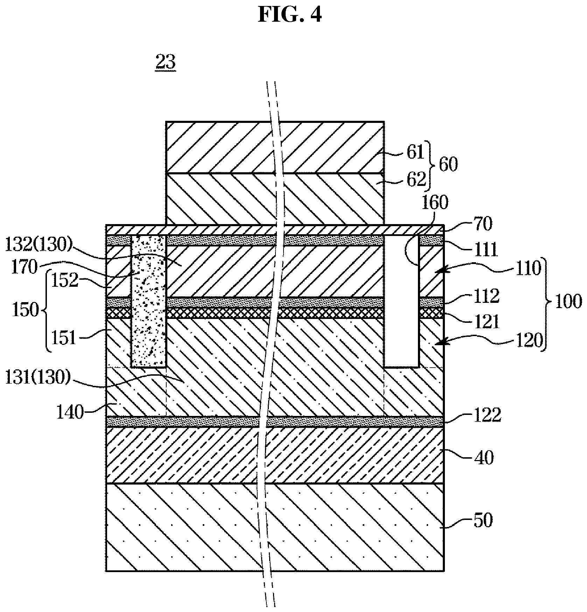

FIG. 4 is an enlarged view of a portion of a transducer in the ultrasonic probe according to an embodiment of the disclosure. As illustrated in FIG. 4, the piezoelectric layer 110 according to an embodiment of the disclosure may include a single crystal.

Although the single crystal may have advantages in that it may implement excellent transmission / reception sensitivity and broadband characteristics compared with the polycrystalline material, the single crystal is expensive compared to the polycrystalline materials and vulnerable to external impact, which may cause cutting damage during machining, resulting in poor quality of the product.

Generally, when a single crystal is processed by a cutting machine to process the single crystal to a size corresponding to the piezoelectric layer 110 for use in the ultrasonic probe 20 (refer to FIG. 3), the single crystal is processed in a single product state, so that the frequency of cutting damage occurring in the single crystal may be increased.

In the transducer 23 according to an embodiment of the disclosure, the single crystal constituting the piezoelectric layer 110 may be processed in a state of being bonded to the acoustic layer 120, rather than being processed in a single product state.

The acoustic layer 120 may include a tungsten carbide, and the acoustic layer 120 having a high hardness firmly adheres and fixes the piezoelectric layer 110, so that the cutting damage of the single crystal may be reduced.

In addition, the acoustic layer 120 may prevent shaking of a dicing blade that dices the piezoelectric layer 110 with high hardness.

To this end, the transducer layer 100 according to an embodiment of the disclosure may include an active portion 130 configured to transmit and receive ultrasonic waves, and a stepped portion 140 extending outwardly from the active portion 130 to prevent cutting damage of the active portion 130.

The transducer 23 may be configured in one dimension in a plurality of arrangements. In general, a direction in which the transducer 23 is divided and arranged into a plurality of portions may be referred to as an azimuth direction, and a width direction of the transducer 23 orthogonal to the divided and arranged direction may be referred to as an elevation direction.

The stepped portion 140 may extend from the active portion 130 in the azimuth direction. The stepped portion 140 may extend toward an edge of the case 21 (refer to FIG. 3) from the active portion 130.

The stepped portion 140 may be formed by dicing the acoustic layer 120 bonded to the piezoelectric layer 110 together with the piezoelectric layer 110 by the dicing blade that dices the piezoelectric layer 110.

The active portion 130 may include a first active portion 131 constituting the acoustic layer 120 and a second active portion 132 constituting the piezoelectric layer 110. The second active portion 132 may be disposed above the first active portion 131.

The acoustic layer 120 may include the first active portion 131 and the stepped portion 140. The stepped portion 140 may extend outwardly from the first active portion 131.

The stepped portion 140 may be stepped with the first active portion 131. The height of the stepped portion 140 and the height of the first active portion 131 may be different. The height of the stepped portion 140 may be lower than the height of the first active portion 131.

The transducer layer 100 may include a damage portion 150 disposed above the stepped portion 140 to protect the active portion 130. The damage portion 150 may extend upward from the stepped portion 140.

The damage portion 150 may include a first damage portion 151 constituting the acoustic layer 120 and a second damage portion 152 constituting the piezoelectric layer 110. The first damage portion 151 may extend upward from the stepped portion 140. The second damage portion 152 may be disposed above the first damage portion 151.

The damage portion 150 may include a portion of the edge of the transducer layer 100 remaining after being diced by the dicing blade that dices the piezoelectric layer 110.

The acoustic layer 120 may include the first active portion 131, the stepped portion 140, and the first damage portion 151. The piezoelectric layer 110 may include the second active portion 132 and the second damage portion 152.

The damage portion 150 may include the same lamination structure as the lamination structure of the transducer layer 100.

That is, the damage portion 150 may include the first electrode 111, the piezoelectric layer 110 disposed below the first electrode 111, the second electrode 112 disposed below the piezoelectric layer 110, the third electrode 121 disposed below the second electrode 112, and the acoustic layer 120 disposed below the third electrode 121.

The transducer layer 100 may include a cut portion 160 formed by the dicing blade that dices the piezoelectric layer 110. The cut portion 160 may be formed near an edge of the transducer layer 100.

FIG. 4 illustrates that two of the cut portions 160 are provided near opposite side edges of the transducer layer 100, but the disclosure is not limited thereto. For example, only the one cut portion 160 may be provided near one side edge of the transducer layer 100.

The cut portion 160 may be formed by the active portion 130, the stepped portion 140, and the damage portion 150. The cut portion 160 may extend from an upper portion of the piezoelectric layer 110 toward the inside of the acoustic layer 120.

That is, the cut portion 160 may be formed by dicing the piezoelectric layer 110 bonded to the acoustic layer 120 by the dicing blade for dicing the piezoelectric layer 110. The width of the cut portion 160 in the azimuth direction may be 10 *µ*m or more. However, the disclosure is not limited thereto.

The damage portion 150 may be spaced apart and separated from the active portion 130 by the cut portion 160.

That is, the first electrode 111, the second electrode 112, and the third electrode 121, which are disposed on the damage portion 150, may be electrically separated from the second electrode 112, and the third electrode 121, which are disposed on the active portion 130. The first electrode 111, the second electrode 112, and the third electrode 121 may not be disposed on the cut portion 160.

The second electrode 112 and the third electrode 121, which are disposed on the damage portion 150, may be electrically connected to the second electrode 112 and the third electrode 121 which are disposed on the active portion 130 through the acoustic layer 120.

That is, the second electrode 112 and the third electrode 121 which are disposed on the damage portion 150 and the second electrode 112 and the third electrode 121, which are disposed on the active portion 130, may be electrically connected through the stepped portion 140.

By disposing the damage portion 150 at an outer side of the cut portion 160, the transducer layer 100 according to an embodiment of the disclosure may protect the active portion 130 from external impact.

That is, the single crystal having been diced for use in the piezoelectric layer 110 may have a significant cutting damage at a diced edge portion. Therefore, by forming the cut portion 160 by the dicing blade, a portion where cutting damage largely occurs may be separated from the active portion 130.

When the cut portion 160 is formed by the dicing blade, the portion where the cutting damage largely occurs may constitute the damage portion 150.

By maintaining the damage portion 150 without removing it completely in the dicing process for forming the cut portion 160, the active portion 130 may be protected from additional external impact that may occur when using the manufactured ultrasonic probe 20 (refer to FIG. 3).

Particularly, when the transducer layer 100 is diced to form the general cut portion 160, only the cutting damage of the upper and lower portions of the second active portion 132 may be prevented, but side cutting damage of a side of the second active portion 132 as well as the cutting damage of the upper and lower portions of the second active portion 132 may be prevented by the damage portion 150.

Also, the transducer layer 100 according to an embodiment of the disclosure includes the stepped portion 140, the damage portion 150, and the cut portion 160, so that the cutting damage of the active portion 130 may be prevented in an additional process of dicing the transducer layer 100 in the elevation direction.

The ground electrode 70 may cover an upper end of the active portion 130, the cut portion 160, and the damage portion 150.

The width of the matching layer 60 in the azimuth direction may correspond to the width of the active portion 130 in the azimuth direction. The width of the matching layer 60 in the azimuth direction may be the same as the width of the active portion 130 in the azimuth direction. However, the disclosure is not limited thereto.

The transducer 23 may include an anti-vibration member 170 that covers an outer side surface of the active portion 130 to protect the active portion 130 and is disposed on an upper portion of the stepped portion 140.

The anti-vibration member 170 may have a hardness higher than that of the piezoelectric layer 110. The piezoelectric layer 110 and the acoustic layer 120 may be bonded together by an adhesive and the anti-vibration member 170 may include a component of the adhesive bonding the piezoelectric layer 110 and the acoustic layer 120.

For example, the anti-vibration member 170 may include epoxy resin, but the disclosure is not limited thereto.

The anti-vibration member 170 may be received in the cut portion 160. Although FIG. 4 illustrates a configuration in which the one anti-vibration member 170 is received in only one of the two cut portions 160 formed near the opposite side edges of the transducer layer 100, the disclosure is not limited thereto.

For example, two of the anti-vibration members 170 may be provided to be received in both of the two cut portions 160 formed near the opposite side edges of the transducer layer 100.

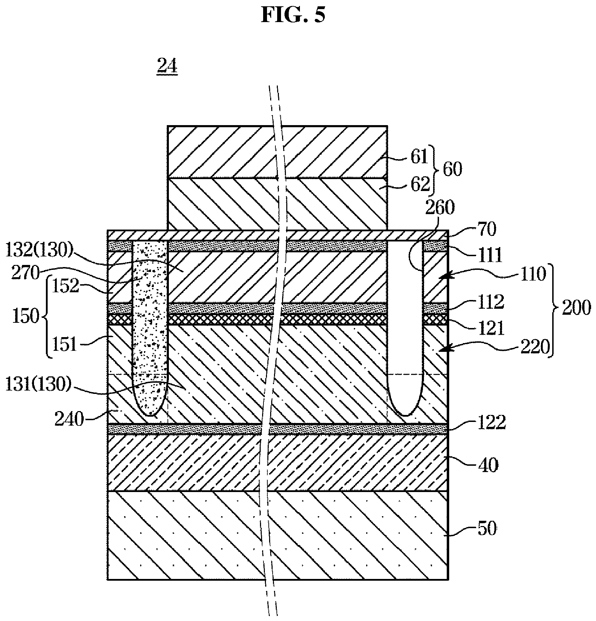

FIG. 5 is an enlarged view of a portion of a transducer in an ultrasonic probe according to another embodiment of the disclosure.

As illustrated in FIG. 5, a transducer 24 according to another embodiment of the disclosure may include substantially the same configuration as the transducer 23 (refer to FIG. 4) according to an embodiment of the disclosure and may differ from the transducer 23 only in the structure of a stepped portion 240 and a cut portion 260.

Hereinafter, the structure of the transducer 24 according to another embodiment of the disclosure will be described, focusing on the differences.

A transducer layer 200 may include the piezoelectric layer 110 and an acoustic layer 220 bonded to a lower end of the piezoelectric layer 110.

The transducer layer 200 may include the stepped portion 240 extending outwardly from the active portion 130 to prevent cutting damage of the active portion 130.

The transducer layer 200 may include the cut portion 260. The transducer 24 may include an anti-vibration member 270 that covers an outer side surface of the active portion 130 to protect the active portion 130 and is disposed on an upper portion of the stepped portion 240. The anti-vibration member 270 may be received in the cut portion 260.

The stepped portion 240 and the cut portion 260 of the transducer 24 according to another embodiment of the disclosure may have a curvature. The anti-vibration member 270 received in the cut portion 260 may have a curvature. The curvature of the stepped portion 240 and the cut portion 260 may be formed according to the shape of a dicing blade that dices the transducer layer 200.

FIG. 5 illustrates that one end portion of the stepped portion 240 and the cut portion 260 have a substantially U shape, but the disclosure is not limited thereto. That is, the one end portion of the stepped portion 240 and the cut portion 260 may be variously formed according to the shape of the dicing blade that dices the transducer layer 200.

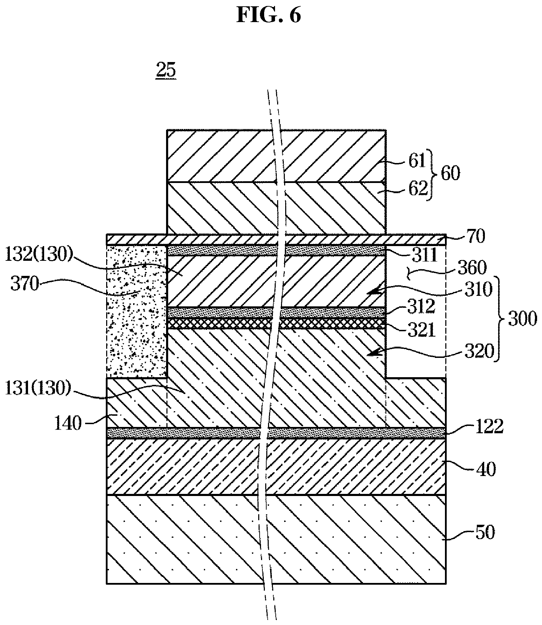

FIG. 6 is an enlarged view of a portion of a transducer in an ultrasonic probe according to another embodiment of the disclosure.

As illustrated in FIG. 6, a transducer 25 according to another embodiment of the disclosure may include substantially the same configuration as the transducer 23 (refer to FIG. 4) according to an embodiment of the disclosure and may differ from the transducer 23 only in the structure of the damage portion 150 and a cut portion 360.

Hereinafter, the structure of the transducer 25 according to another embodiment of the disclosure will be described, focusing on the differences.

A transducer layer 300 may include the cut portion 360. The cut portion 360 may be formed at an edge of the transducer layer 300.

FIG. 6 illustrates that two of the cut portions 360 are provided at opposite side edges of the transducer layer 300, but the disclosure is not limited thereto. For example, only the one cut portion 360 may be provided at one side edge of the transducer layer 300.

The cut portion 360 may be formed by the active portion 130 and the stepped portion 140. The cut portion 360 may extend from a piezoelectric layer 310 toward an acoustic layer 320.

That is, the cut portion 360 may be formed by dicing the piezoelectric layer 310 bonded to the acoustic layer 320 by a dicing blade that dices the piezoelectric layer 310. The width of the cut portion 360 in the azimuth direction may be 10 *µ*m or more. However, the disclosure is not limited thereto.

The cut portion 360 may include a space provided at an outer edge of the active portion 130 and at an upper portion of the stepped portion 140. The transducer layer 300 according to another embodiment of the disclosure may prevent cutting damage of the upper and lower portions of the second active portion 132 by the cut portion 360.

The transducer 25 according to another embodiment of the disclosure may not include the damage portion 150 (refer to FIG. 4) extending from the stepped portion 140, unlike the transducer 23 according to an embodiment of the disclosure.

The single crystal having been diced for use in the piezoelectric layer 310 may have a significant cutting damage at a diced edge portion. The portion where the cutting damage largely occurs may correspond to the edge portion of the diced transducer layer 300 to form the cut portion 360 and may be removed in the process of forming the cut portion 360.

The process of forming the cut portion 360 may be simplified by completely removing the damage portion 150 by the dicing process for forming the cut portion 360.

The ground electrode 70 may cover the upper end of the active portion 130 and the cut portion 360. That is, the ground electrode 70 may cover an upper end of the piezoelectric layer 310.

The transducer 25 may include an anti-vibration member 370 that covers an outer side surface of the active portion 130 to protect the active portion 130 and is disposed on an upper portion of the stepped portion 140. The anti-vibration member 370 may be received in the cut portion 360.

The anti-vibration member 370 may be received between the active portion 130 and the case 21 (refer to FIG. 3). FIG. 6 illustrates that the anti-vibration member 370 is formed in a substantially rectangular shape, but the disclosure is not limited thereto. That is, the anti-vibration member 370 may include various shapes corresponding to the shape of the cut portion 360 formed between the active portion 130, the stepped portion 140 and the case 21.

A first electrode 311 connected to the ground electrode 70 may be provided on the front surface of the piezoelectric layer 310 and a second electrode 312 electrically connected to the signal electrode 40 may be provided on the rear surface of the piezoelectric layer 310.

The acoustic layer 320 may include a third electrode 321 and the fourth electrode 122. The third electrode 321 may be formed on the front surface of the acoustic layer 320 and the fourth electrode 122 may be formed on the rear surface of the acoustic layer 320. The third electrode 321 and the acoustic layer 320 may be electrically connected.

The first electrode 311, the second electrode 312, and the third electrode 321 may be electrically connected without being disconnected from each other regardless of the cut portion 360.

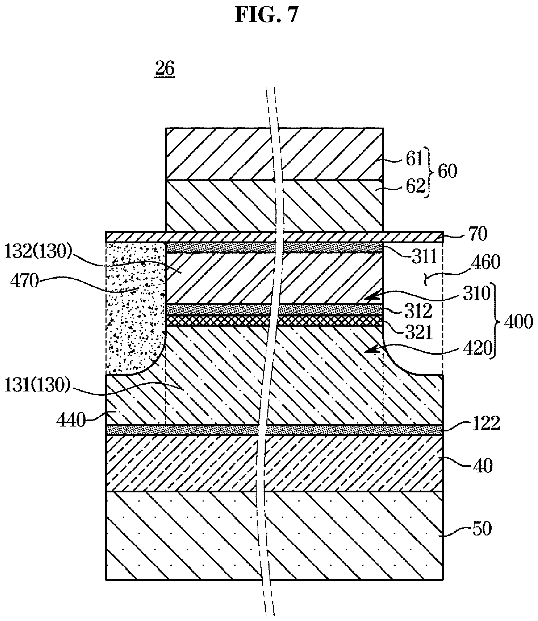

FIG. 7 is an enlarged view of a portion of a transducer in an ultrasonic probe according to another embodiment of the disclosure.

As illustrated in FIG. 7, a transducer 26 according to another embodiment of the disclosure may include substantially the same configuration as the transducer 25 (refer to FIG. 6) according to another embodiment of the disclosure and may differ from the transducer 25 only in the structure of a stepped portion 440 and a cut portion 460.

Hereinafter, the structure of the transducer 26 according to another embodiment of the disclosure will be described, focusing on the differences.

A transducer layer 400 may include the stepped portion 440 extending outwardly from the active portion 130 to prevent cutting damage to the active portion 130.

An acoustic layer 420 may include the cut portion 460. The cut portion 460 may be formed at an edge of the transducer layer 400.

The transducer 26 may include an anti-vibration member 470 that covers an outer side surface of the active portion 130 to protect the active portion 130 and is disposed on an upper portion of the stepped portion 440. The anti-vibration member 470 may be received in the cut portion 460.

The stepped portion 440 and the cut portion 460 of the transducer 26 according to another embodiment of the disclosure may have a curvature. The anti-vibration member 470 received in the cut portion 460 may have a curvature. The curvature of the stepped portion 440 and the cut portion 460 may be formed according to the shape of a dicing blade that dices the transducer layer 400.

FIG. 7 illustrates that one end portion of the stepped portion 440 and the cut portion 460 have a substantially arc shape, but the disclosure is not limited thereto. That is, the one end portion of the stepped portion 440 and the cut portion 460 may be variously formed according to the shape of the dicing blade that dices the transducer layer 400.

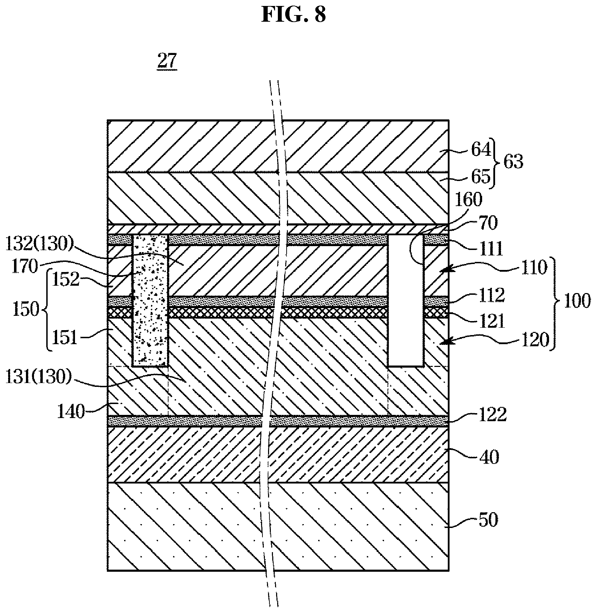

FIG. 8 is an enlarged view of a portion of a transducer in an ultrasonic probe according to another embodiment of the disclosure.

As illustrated in FIG. 8, a transducer 27 according to another embodiment of the disclosure may include substantially the same configuration as the transducer 23 (refer to FIG. 4) according to an embodiment of the disclosure and may differ from the transducer 23 only in the structure of a matching layer 63.

Hereinafter, the structure of the transducer 27 according to another embodiment of the disclosure will be described, focusing on the difference.

The transducer 27 according to another embodiment of the disclosure may include the matching layer 63. The matching layer 63 may be disposed to be adjacent to the piezoelectric layer 110.

A plurality of the matching layers 63 may be provided to be laminated so that the acoustic impedance may gradually change from the piezoelectric layer 110 toward the target object. The matching layer 63 may include a first matching layer 64 and a second matching layer 65. The plurality of matching layers 63 may be provided to have different materials.

The width of the matching layer 63 in the azimuth direction may correspond to the width of the transducer layer 100 in the azimuth direction. The width of the matching layer 63 in the azimuth direction may be the same as the width of the transducer layer 100 in the azimuth direction. However, the disclosure is not limited thereto.

That is, the matching layer 63 of the transducer 27 according to another embodiment of the disclosure may have a width larger than the width of the active portion 130 so as to cover the cut portion 160 and the damage portion 150.

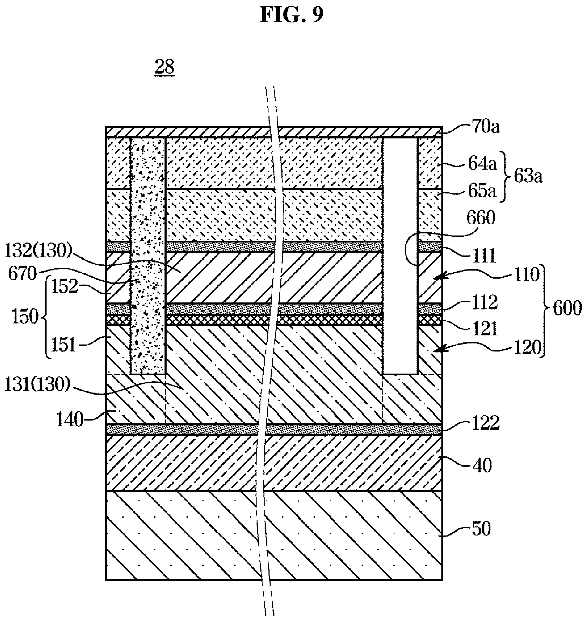

FIG. 9 is an enlarged view of a portion of a transducer in an ultrasonic probe according to another embodiment of the disclosure.

As illustrated in FIG. 9, a transducer 28 according to another embodiment of the disclosure may include substantially the same configuration as the transducer 27 (refer to FIG. 8) according to another embodiment of the disclosure and may differ from the transducer 27 only in the structure of a matching layer 63a and a cut portion 660.

Hereinafter, the structure of the transducer 28 according to another embodiment of the disclosure will be described, focusing on the differences.

The transducer 28 according to another embodiment of the disclosure may include the matching layer 63a. The matching layer 63a may be disposed to be adjacent to the piezoelectric layer 110.

A plurality of the matching layers 63a may be provided to be laminated so that the acoustic impedance may gradually change from the piezoelectric layer 110 toward the target object. The matching layer 63a may include a first matching layer 64a and a second matching layer 65a. The plurality of matching layers 63a may be provided to have different materials.

The matching layer 63a of the transducer 28 according to another embodiment of the disclosure may include a conductive matching layer. A transducer layer 600 may include the cut portion 660. The cut portion 660 may be formed near an edge of the transducer layer 600.

The cut portion 660 may be formed by the active portion 130, the stepped portion 140, the damage portion 150 and the matching layer 63a. The cut portion 660 may extend from an upper end of the first matching layer 64a toward the inside of the acoustic layer 120. That is, the cut portion 660 may extend to an upper end of the matching layer 63a.

The stepped portion 660 may be formed by dicing the piezoelectric layer 110 bonded between the acoustic layer 120 and the matching layer 63a by the dicing blade for dicing the piezoelectric layer 110. Therefore, the single crystal may be processed more stably by the transducer 28 according to another embodiment of the disclosure.

A ground electrode 70a may cover the upper end of the matching layer 63a and the cut portion 660.

The transducer 28 may include an anti-vibration member 670 that covers an outer side surface of the active portion 130 to protect the active portion 130 and is disposed on an upper portion of the stepped portion 140. The anti-vibration member 670 may be received in the cut portion 660.

FIG. 9 illustrates that the first matching layer 64a and the second matching layer 65a both include a conductive matching layer, but the disclosure is not limited thereto, and at least one of the first matching layer 64a and the second matching layer 65a may include a conductive matching layer.

For example, the first matching layer 64a may be formed of a general matching layer, and only the second matching layer 65a disposed below the first matching layer 64a may include a conductive matching layer.

In this case, the cut portion 660 may be formed by the active portion 130, the stepped portion 140, the damage portion 150 and the matching layer 63a, and may extend from an upper end of the second matching layer 65a toward the inside of the acoustic layer 120.

Also, the cut portion 660 may extend only to the upper end of the second matching layer 65a, and the ground electrode 70a may be disposed between the first matching layer 64a and the second matching layer 65a to cover the upper end of the second matching layer 65a and the cut portion 660.

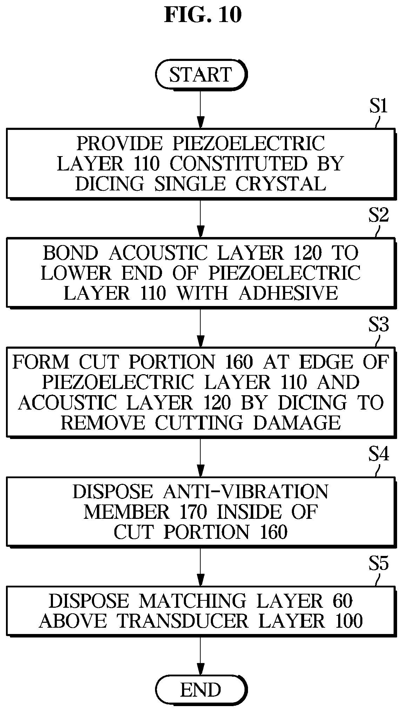

FIG. 10 is a flow chart for manufacturing a part of a transducer in the ultrasonic probe according to an embodiment of the disclosure. A method of manufacturing the transducer 23 (refer to FIG. 3) of the ultrasonic probe 20 (refer to FIG. 3) according to an embodiment of the disclosure will be briefly described with reference to FIG. 10.

In order to manufacture the transducer 23, the piezoelectric layer 110 formed by dicing a single crystal to generate ultrasonic waves may be provided (S1), and the acoustic layer 120 may be bonded to a lower end of the piezoelectric layer 110 through an adhesive (S2).

The cut portion 160 from the upper end of the piezoelectric layer 110 toward the inside of the acoustic layer 120 may be formed near an edge of the piezoelectric layer 110 and the acoustic layer 120 by an additional dicing process so that cutting damage occurring at an edge of the piezoelectric layer 110 in the process of dicing the single crystal is removed (S3).

Through this dicing process, the damage portion 150 provided at an edge of the transducer layer 100 (refer to FIG. 4) may be maintained or removed, and the cut portion 160 may be formed to correspond to the shape of the dicing blade.

The anti-vibration member 170 may be disposed inside the cut portion 160 to protect the active portion 130 (refer to FIG. 4) constituting an inner side surface of the cut portion 160 (S4).

Only the active portion 130 may be covered or all of the active portion 130, the cut portion 160 and the damage portion 150 may be covered by positioning the matching layer 60 on an upper end of the transducer layer 100 (S5).

A method of manufacturing the ultrasonic probe 20 according to an embodiment of the disclosure may include dicing a single crystal first for use as the piezoelectric layer 110, and forming the cut portion 160 near the edge of the transducer layer 100 through additional dicing after bonding the acoustic layer 120 and the piezoelectric layer 110 in order to remove the cutting damage of the diced single crystal.

However, the disclosure is not limited thereto, and the method according to an embodiment of the disclosure, before the dicing process for using the single crystal as the piezoelectric layer 110, may include bonding the single crystal to a raw material of the acoustic layer 120 and then dicing the single crystal bonded to the raw material of the acoustic layer 120, so that cutting damage that may occur in the single crystal may be prevented in advance.

As is apparent from the above, the disclosure can improve the reliability in the quality of an ultrasonic probe by preventing the aging failure of the ultrasonic probe.

The disclosure can use a single crystal as a piezoelectric material forming a piezoelectric layer.

The disclosure can prevent cutting damage that may occur in a single crystal forming a piezoelectric layer.

Although the technical idea of the disclosure has been described above with reference to specific embodiments, the scope of the disclosure is not limited to these embodiments.

It will be understood by those skilled in the art that various changes in form and details may be made therein without departing from the spirit and scope of the disclosure as defined by the appended claims.

## Claims

1. An ultrasonic probe comprising:
a transducer layer including a piezoelectric layer configured to generate ultrasonic waves and an acoustic layer disposed below the piezoelectric layer; and
a matching layer disposed above the piezoelectric layer,
wherein the transducer layer includes an active portion configured to transmit and receive ultrasonic waves, and a stepped portion extending outward from the active portion to prevent cutting damage of the active portion.

2. The ultrasonic probe according to claim 1, wherein
the stepped portion extends from the active portion in an azimuth direction.

3. The ultrasonic probe according to claim 1, wherein
the piezoelectric layer includes a single crystal.

4. The ultrasonic probe according to claim 1, wherein
the acoustic layer includes an acoustic reflector having a tungsten carbide.

5. The ultrasonic probe according to claim 1, wherein
the active portion includes a first active portion constituting the acoustic layer and a second active portion disposed above the first active portion to constitute the piezoelectric layer, and
the stepped portion is stepped with the first active portion.

6. The ultrasonic probe according to claim 5, wherein
the height of the stepped portion is lower than the height of the first active portion.

7. The ultrasonic probe according to claim 1, further comprising:
an anti-vibration member covering an outer side surface of the active portion to protect the active portion and disposed on an upper portion of the stepped portion.

8. The ultrasonic probe according to claim 7, wherein
the anti-vibration member has a hardness higher than the hardness of the piezoelectric layer.

9. The ultrasonic probe according to claim 7, wherein
the piezoelectric layer and the acoustic layer are bonded by an adhesive, and
the anti-vibration member includes a component of the adhesive.

10. The ultrasonic probe according to claim 7, wherein
the transducer layer further includes a damage portion disposed above the stepped portion to prevent cutting damage of the active portion.

11. The ultrasonic probe according to claim 10, wherein
the damage portion is spaced apart from the outer side surface of the active portion to form a cut portion together with the active portion and the stepped portion, and
the anti-vibration member is received in the cut portion.

12. The ultrasonic probe according to claim 10, wherein
the damage portion includes a first damage portion extending upward from the stepped portion to constitute the acoustic layer and a second damage portion disposed above the first damage portion to constitute the piezoelectric layer.

13. The ultrasonic probe according to claim 1, wherein
the stepped portion has a curvature.

14. The ultrasonic probe according to claim 11, wherein
the matching layer has a width larger than the width of the active portion to cover the cut portion and the damage portion.

15. A method of manufacturing an ultrasonic probe comprising:
providing a piezoelectric layer configured to generate ultrasonic waves;
bonding an acoustic layer to a lower end of the piezoelectric layer by an adhesive; and
forming a cut portion toward the inside of the acoustic layer from an upper end of the piezoelectric layer near an edge of the piezoelectric layer and the acoustic layer so that the edge of the piezoelectric layer damaged by cutting damage is removed.
